# FASCICULE DE BREVET EUROPEEN

(11) **EP 4 487 812 B1**
(45) Date de publication et mention de la délivrance du brevet: **09.12.2026**
(21) Numéro de dépôt: 24186517.9
(22) Date de dépôt: 04.07.2024
(51) Int. Cl.: A61C 8/02, A61C 8/00, A61F 2/28

(54) **NOUVEAU SYSTÈME DE RÉGÉNÉRATION OSSEUSE**
NEUES KNOCHENREGENERATIONSSYSTEM
NOVEL BONE REGENERATION SYSTEM

(30) Priorité: 07.07.2023 FR 2307269
(43) Date de publication de la demande: 08.01.2025
(73) Titulaire: BIOTECH DENTAL, 13300 Salon-de-Provence (FR); The Bone Institute, 06000 Nice (FR)
(72) Inventeur: SURMENIAN, Jérôme, 06000 Nice (FR)
(74) Mandataire: Hautier IP

(56) Documents cités:
- WO-A1-2012/134006
- FR-A1- 3 072 563
- KR-A- 20100 025 369
- US-A1- 2006 154 205
- US-B2- 9 877 807

## Description

La présente demande de brevet revendique la priorité de la demande de brevet Français FR 2307269 déposée en date du 7 juillet 2024.

### DOMAINE DE L'INVENTION

La présente invention est relative au domaine des dispositifs destinés à la régénération osseuse et, plus spécifiquement, à celui de la stabilisation des implants dentaires endo-osseux.

### ART ANTERIEUR

Classiquement, les implants endo-osseux prennent la forme d'un pied d'ancrage adapté pour une implantation dans un os de la mâchoire d'un patient, lequel pied d'ancrage correspond le plus souvent à une vis d'ancrage.

Un exemple de tels implants est donné dans la demande de brevet US 2003/232308 dans lequel un pied d'ancrage (base) destiné à être positionné dans un logement de forme complémentaire préparé dans l'os de la mâchoire du patient présente également une collerette (plateforme) s'étendant à partir de la base et avec laquelle elle forme donc une seule et même pièce. Maintenant, ce pied d'ancrage coopère le plus souvent avec un pilier externe qui est destiné à être fixé dans le pied d'ancrage et qui va être couplé à une connexion prothétique permettant de fixer une couronne pour remplacer l'élément dentaire manquant.

US 2006/154205 A1 divulgue un un autre exemple d'implant endo-osseux destiné à assurer le maintien d'un substitut de régénération osseuse.

Maintenant, une telle implantation peut s'avérer compliqué par la présence de défauts osseux au sein même de la mâchoire du patient. De tels défauts osseux, qui peuvent avoir de multiples origines (infectieuse, tumorale ou traumatique), résultent d'un défaut de cicatrisation osseuse et correspondent à des cavités en lieu et place de l'os détruit.

Face à de tels défauts osseux, il est possible d'utiliser des biomatériaux permettant tout à la fois le comblement du défaut osseux et de favoriser la cicatrisation osseuse en son sein.

En règle générale, le biomatériau utilisé est constitué soit de matériau de substitution osseuse synthétique (par exemple des granules d'hydroxyapatite), soit de particules d'os. Une fois l'évidement correspondant au défaut osseux rempli par ce biomatériau, cet évidement est fermé par une membrane ou une plaque de recouvrement qui permet de diriger et contenir la croissance de ce biomatériau, on parle de Régénération Osseuse Guidée (ROG). A titre d'exemple de telles membranes et plaques de recouvrement, on peut citer celles décrites dans le brevet US 4,816,339 et dans le brevet EP 2 536 446 B1 respectivement.

Chacune de ces solutions (membrane ou plaque de recouvrement) présentent ses avantages et inconvénients respectifs.

La membrane de recouvrement, par sa structure, permet le passage des substances nutritives permettant la régénération osseuse tout en n'étant pas colonisée par les cellules osseuses. Aussi, la membrane de recouvrement peut être simplement retirée une fois la régénération terminée sans risquer de fragiliser ou d'entraîner de l'os régénéré. Maintenant, la nature « souple » de cette membrane de recouvrement permet difficilement d'encadrer le volume de régénération osseuse.

A l'inverse, la plaque de recouvrement, de par sa rigidité, permet de guider de façon optimale le volume de cette régénération. Maintenant, la perforation de sa structure pour permettre le passage des substances nutritives vers le site de régénération entraine sa colonisation par les cellules osseuses. Il en résulte que le retrait de la plaque de recouvrement au terme de la régénération est compliqué et est susceptible d'affecter l'intégrité de l'os régénéré.

Pour ce qui est du maintien de la membrane ou plaque de recouvrement, il est assuré par des vis fixées à l'os entourant le défaut osseux.

Maintenant, et dans le cas d'un défaut osseux de taille modérée, le praticien utilisera en général une membrane de recouvrement. Dans ce cas, le maintien de celle-ci pourra être assuré par une vis se fixant au pied d'ancrage du futur implant dentaire prépositionné au sein même du défaut osseux de la mâchoire du patient. En revanche, le praticien n'utilisera pas de plaque de recouvrement dans un tel cas, sachant qu'elles nécessitent des points de fixation multiples dont le traumatisme pour l'os, déjà affaibli, de la mâchoire du patient ne se justifierait pas au regard de la taille modérée du défaut osseux à traiter. Le traumatisme résultant va en effet entrainer des défauts osseux qui pourraient être plus important que celui dont la régénération est envisagée.

Aussi, il importe de développer de nouvelles solutions de régénération osseuse, adaptées spécifiquement à ces défauts osseux de taille modérée, qui présentent une mise en œuvre simplifiée pour le praticien, un traumatisme minimum pour l'os de la mâchoire du patient, tout en assurant le meilleur résultat possible en termes de régénération osseuse au site de défaut osseux.

### SOMMAIRE DE L'INVENTION

Face à cette problématique, l'inventeur a maintenant développé une nouvelle solution adaptée à la régénération osseuse de défaut osseux de taille modérée qui, en dehors de la fixation associée au pied d'ancrage de l'implant à venir, ne nécessite aucune fixation dans l'os entourant le défaut osseux, tout en encadrant le volume de régénération osseuse de façon améliorée. Cette solution particulièrement innovante peut utiliser, ou non, une membrane de recouvrement.

Dans le détail, l' objet de l'invention porte sur un kit de régénération osseuse pour un site de défaut osseux dans un os de mâchoire d'un patient défini dans la revendication 1. Les modes de réalisation préférés sont définis dans les revendications dépendantes. Le kit comprend :
A) un substitut de régénération osseuse permettant le comblement du défaut osseux et
B) un système de régénération osseuse comprenant :
   1) au moins un pied d'ancrage adapté à une installation de manière endo-osseuse dans l'os de mâchoire, de préférence au sein même du défaut osseux ;
   2) éventuellement au moins une membrane de recouvrement, de préférence une membrane de recouvrement dont la surface permet de couvrir au moins la totalité du défaut osseux comblé par le substitut de régénération osseuse;
   3) au moins un dispositif destiné à assurer le maintien du substitut de régénération osseuse et, éventuellement, de la membrane de recouvrement sur ledit défaut osseux qui comprend :
      3a) sur sa face dirigée vers l'os, au moins un pilier externe destiné à être fixé dans le pied d'ancrage, lequel pilier externe comprend des moyens anti-rotationnels permettant d'inhiber les mouvements relatifs du pilier externe par rapport au pied d'ancrage une fois le pilier externe placé dans le pied d'ancrage ; et
      3b) sur sa face opposée à l'os, au moins une pièce de recouvrement se plaçant sur le substitut de régénération osseuse et, éventuellement, sur la membrane de recouvrement de sorte à délimiter, en combinaison avec ledit substitut de régénération osseuse et, éventuellement, ladite membrane de recouvrement, le volume de la régénération osseuse au niveau dudit site de défaut osseux, laquelle pièce de recouvrement présente :
         3bi) une partie centrale, dans le prolongement du pilier externe, et
         3bii) au moins 2 extensions latérales longilignes (7a, 7b) dans le prolongement de cette i) partie centrale dont l'axe recoupe perpendiculairement l'axe central (AA), correspondant à l'axe traversant la partie centrale et le 1) au moins un pilier d'ancrage, de préférence chaque extension latérale longiligne (7a, 7b) ne comprend aucun orifice destiné au positionnement d'une vis de fixation et/ou lesdites extensions latérales longilignes (7a, 7b) sont flexibles.

Un système de régénération osseuse est décrit précédemment.

Un dispositif destiné au maintien du substitut de régénération osseuse et, éventuellement, de la membrane de recouvrement tel que défini précédemment est également décrit.

Selon un mode de réalisation préféré, le dispositif destiné à assurer le maintien du substitut de régénération osseuse et, éventuellement, de la membrane de recouvrement sur le défaut osseux comprend 3c) un pilier intermédiaire, entre 3a) le pilier externe et 3b) la pièce de recouvrement.

Selon un autre mode de réalisation préféré, le système selon l'invention comprend en outre :
- et pour le 3) au moins un dispositif assurant le maintien du substitut de régénération osseuse et, éventuellement, de la membrane de recouvrement, 3d) au moins une vis qui présente, à son extrémité distale, un filetage externe, et ménagée au sein du 3a) au moins un pilier externe et, s'il est présent, au sein du 3c) au moins un pilier intermédiaire, une cavité interne permettant le passage de ladite 3d) au moins une vis mais pas de sa tête ; et
- au sein du 1) au moins un pied d'ancrage, une cavité dans laquelle est ménagée un taraudage qui peut coopérer avec le filetage de la d) au moins une vis pour permettre l'assemblage desdits 1) au moins un pied d'ancrage et 3) au moins un dispositif assurant le maintien du substitut de régénération osseuse et, éventuellement, de la membrane de recouvrement par vissage du filetage de la 3d) au moins une vis dans le taraudage de la cavité interne du 1) au moins un pied d'ancrage.

Selon encore un autre mode de réalisation préféré, 3a) la pièce de recouvrement forme une seule et même pièce avec 3b) le pilier externe et, éventuellement, 3c) le pilier intermédiaire.

Selon un mode de réalisation préféré complémentaire, le système selon l'invention comprend en outre 4) au moins une pièce qui est complémentaire du 3) au moins un dispositif destiné à assurer le maintien de la membrane de recouvrement sur un défaut osseux, laquelle 3) au moins une pièce complémentaire permet d'en faciliter la préhension par le praticien lors de la mise en place de celui-ci.

Une méthode de régénération osseuse pour un site de défaut osseux dans un os de mâchoire d'un patient, est décrite, mais ne fait pas partie de l'invention revendiquée. Cette laquelle méthode comprend les étapes de :
1) pose d'un pied d'ancrage au sein de l'os de la mâchoire du patient au niveau ou à proximité du défaut osseux ;
2) comblement du défaut osseux avec un substitut de régénération osseuse ;
3) éventuellement positionnement, sur ce substitut de régénération osseuse, d'une membrane de recouvrement ;
4) pose, de préférence sur cette membrane de recouvrement, d'un dispositif destiné au maintien du substitut de régénération osseuse et, éventuellement, de la membrane de recouvrement tel que défini précédemment.

### DESCRIPTION DES FIGURES

La figure 1 est une vue en coupe de l'implantation dans l'os de la mâchoire d'un patient d'un mode de réalisation du système de régénération osseuse selon l'invention.
La figure 2 est une vue en coupe de ce même mode de réalisation du système de régénération osseuse selon l'invention.
La figure 3, la figure 4, la figure 5 et la figure 6 montrent des vues schématiques sous différents angles d'un mode de réalisation d'un dispositif destiné au maintien du substitut de régénération osseuse selon l'invention.

### DESCRIPTION DETAILLEE DE L'INVENTION

Les substituts de régénération osseuse sont bien connus de l'homme du métier et un tel substitut peut être choisi parmi ceux utilisés pour le comblement de perte de substance osseuse dans diverses pathologies. Un tel substitut de régénération osseuse peut être constitué d'un matériau de substitution osseuse synthétique (par exemple des granules d'hydroxyapatite), mais aussi de particules d'os. A titre d'exemple de substituts de régénération osseuse, on peut citer RE-BONE, BIO-OSS, BIOSORB-DENTAL, CREOS, ADBONE, TEEBONE, SYBONE, etc.

En ce qui concerne les pieds d'ancrage, ceux-ci sont également bien connus des praticiens et prennent typiquement la forme d'une vis apte à être vissée dans un logement complémentaire préparé et conformé dans l'os maxillaire du patient. L'os de la mâchoire du patient est ainsi taraudé de sorte à obtenir un filetage qui permettra ensuite la fixation du pied d'ancrage. La vis du pied d'ancrage pourra être de forme cylindrique ou conique.

Le matériau de ce pied d'ancrage est lui avantageusement compatible avec le matériau biologique de l'os de sorte à faciliter son ostéo-intégration dans la mâchoire (os maxillaire et mandibulaire). Typiquement, ce pied d'ancrage pourra être réalisé en zircone (céramique), en titane ou encore en alliage de titane.

En termes de dimensions, un tel pied d'ancrage a :
- une longueur comprise entre 0,2 et 1,5 cm, de préférence entre 0,5 et 0,8 cm ; et
- une section comprise entre 3 et 8 mm, de préférence entre 3 et 6 mm.

Les membranes de recouvrement sont utilisées de longue date dans la Régénération Osseuse Guidée (ROG) et sont, de fait, bien connues des praticiens. Elles peuvent être de nature résorbable ou non résorbable.

Les membranes de recouvrement utilisées classiquement sont non-résorbables et sont réalisées à partir de ePTFE (Polytétrafluoroéthylène expansé) avec la possibilité d'un renforment mécanique avec une armature métallique ; typiquement en titane (ePTFE TR pour Titane Renforcées).

Les membranes résorbables elles, peuvent être soit synthétiques, soit collagéniques.

Les membranes synthétiques sont typiquement constituées de copolymères d'acide polylactique et polyglycolique c'est pourquoi elles se résorbent par hydrolyse. On peut citer à titre d'exemples de telles membranes synthétiques, les membranes commercialisées sous les dénominations ATRISORB, VICRYL, RESOLUT TM/RESOLUT XT.

Les membranes collagéniques ont été utilisées pour la première fois en 1996 et sont fabriquées à partir de collagène réticulé ou non qui est d'origine bovine ou porcine avec des fibres réticulées ou non. On peut citer à titre d'exemples de telles membranes collagéniques, les membranes commercialisées sous les dénominations AVITENE, PAROGUIDE, BIO-GUIDE, PERIOGEN ou encore BIOMEND.

Les dimensions de la au moins une membrane de recouvrement sont telles qu'elle doit, de préférence, permettre de couvrir au moins la totalité du défaut osseux.

Typiquement, une telle membrane de recouvrement présente :
- une longueur comprise entre 0,5 et 2,5 cm, de préférence entre 0,5 et 1,5 cm ; et
- une largeur comprise entre 0,5 et 2,5 cm, de préférence entre 0,5 et 1,5 cm.

Le dispositif destiné à assurer le maintien du substitut de régénération osseuse et, éventuellement, de la membrane de recouvrement sur le défaut osseux doit présenter une rigidité suffisante de sorte à assurer sa fonction. Maintenant et préférentiellement, la pièce de recouvrement de ce même dispositif doit présenter simultanément une élasticité qui permet son ajustement, lors de sa pose, par la courbure de ses extensions latérales longilignes, de sorte à délimiter, éventuellement en combinaison avec la membrane de recouvrement, le volume de régénération du défaut osseux.

Pour se faire, le dispositif assurant le maintien du substitut de régénération osseuse et, éventuellement, de la membrane de recouvrement ou, à minima sa pièce de recouvrement, est avantageusement réalisé en titane.

Le titane est un métal de transition brillant avec une faible densité et une résistance élevée. Le titane pur est ainsi plus résistant que les aciers ordinaires à faible teneur en carbone, tout en étant 45 % plus léger. L'intégration du titane au sein de différents alliages permet de leur associer des propriétés particulièrement intéressantes. Ainsi, des alliages de titane sont généralement utilisé pour la plupart des applications avec de petites quantités d'aluminium et de vanadium, généralement 6% et 4% respectivement, en poids. Le titane peut se trouver sous deux formes cristallines (alpha et béta) et, les alliages de titane, en fonction de leur composition, vont orienter le titane vers l'une ou l'autre de ces formes cristallines modifiant par la même les propriétés de l'alliage. Parmi ces alliages, le titane de Grade 1, ou T35, et le titane de Grade 2, ou T40, constitue des alliages spécifiques, dont la composition (en Titane, en Oxygène, en Fer, en Carbone, en Hydrogène et en Azote) et les caractéristiques sont bien connues de l'homme du métier.

Avantageusement, le dispositif assurant le maintien du substitut de régénération osseuse et, éventuellement, de la membrane de recouvrement est réalisée en titane de grade 1 ou de grade 2.

Selon l'invention, le 3) au moins un dispositif destiné à assurer le maintien du A) substitut de régénération osseuse et, éventuellement, de la 2) au moins une membrane de recouvrement sur le défaut osseux comprend, entre le 3a) au moins un pilier externe et la 3b) au moins une pièce de recouvrement, préférablement un 3c) au moins un pilier intermédiaire que l'on pourrait qualifier de partie transmuqueuse.

Idéalement, la hauteur de ce 3c) au moins un pilier intermédiaire est comprise dans l'intervalle allant de 0,5 à 5 mm, de préférence dans l'intervalle allant de 1 à 2 mm.

De préférence, ce 3c) au moins un pilier intermédiaire comprend un étranglement dans le sens de la largeur en allant vers l'os de la mâchoire et peut, éventuellement, également comprendre un feston. Cet étranglement présente idéalement un profil conique, lequel profil conique est dénué de tout angle potentiellement vulnérant.

Le système selon l'invention comprend en outre :
- et pour le 3) au moins un dispositif assurant le maintien du substitut de régénération osseuse et, éventuellement, de la membrane de recouvrement, 3d) au moins une vis qui présente, à son extrémité distale, un filetage externe, et ménagée au sein du a) au moins un pilier externe et, s'il est présent, au sein du 3c) au moins un pilier intermédiaire, une cavité interne permettant le passage de ladite 3d) au moins une vis mais pas de sa tête ; et
- au sein du 1) au moins un pied d'ancrage, une cavité dans laquelle est ménagée un taraudage qui peut coopérer avec le filetage de la 3d) au moins une vis pour permettre l'assemblage desdits 1) au moins un pied d'ancrage et 3) au moins un dispositif assurant le maintien du substitut de régénération osseuse et, éventuellement, de la membrane de recouvrement par vissage du filetage de la 3d) au moins une vis dans le taraudage de la cavité interne du 1) au moins un pied d'ancrage.

Dans ce mode de réalisation, cette 3d) au moins une vis permet l'assemblage par trans-vissage desdits 1) au moins un pied d'ancrage et 3) au moins un dispositif assurant le maintien du substitut de régénération osseuse et, éventuellement, de la membrane de recouvrement.

Avantageusement, la tête de ladite 3d) au moins une vis et le 3a) au moins un pilier externe ou, s'il est présent, le 3c) au moins un pilier intermédiaire présentent des moyens de centrage. Typiquement, ces moyens de centrage sont tels que la tête de ladite d) au moins une vis et le 3a) au moins un pilier externe ou, s'il est présent, le 3c) au moins un pilier intermédiaire comprennent des portions femelle et mâle complémentaires. De préférence, ces formes femelle et mâle complémentaires sont des formes tronconiques d'axe vertical. Dès lors, outre le centrage, ces formes permettent d'inhiber les mouvements relatifs et d'assurer l'étanchéité entre la d) au moins une vis et le a) au moins un pilier externe ou, s'il est présent, le c) au moins un pilier intermédiaire.

De préférence, la d) au moins une vis et le a) au moins un pilier externe ou, s'il est présent, le 3c) au moins un pilier intermédiaire présentent des moyens de centrage complémentaires lesquels sont compris dans la longueur de la 3d) au moins une vis et dans la cavité interne du 3a) au moins un pilier externe ou, s'il est présent, du 3c) au moins un pilier intermédiaire.

Avantageusement encore, un taraudage est ménagé au sein de la cavité interne du a) au moins un pilier externe. Ce taraudage peut coopérer avec un filetage externe présent, par exemple sur un extracteur, de sorte à permettre le désassemblage du 1) au moins un pied d'ancrage du 3) au moins un dispositif destiné à assurer le maintien du substitut de régénération osseuse et, éventuellement, de la membrane de recouvrement sur ledit défaut osseux.

En lien maintenant avec la 3b) au moins une pièce de recouvrement, celle-ci présente 3bi) une partie centrale, dans le prolongement du 3a) au moins un pilier externe ou, s'il est présent, du 3c) au moins un pilier intermédiaire, laquelle 3bi) partie centrale est avantageusement circulaire et prend alors typiquement la forme d'une rondelle.

Cette 3b) au moins une pièce de recouvrement présente en outre, 3bii) au moins 2 extensions latérales longilignes, de préférence 3bii) au moins 3 extensions, lesquelles extensions latérales longilignes sont avantageusement flexibles.

Aussi, la 3bi) partie centrale de la 3b) au moins une pièce de recouvrement constitue la base des 3bii) au moins 2 extensions latérales longilignes, de préférence des 3bii) au moins 3 extensions latérales longilignes.

Les extensions latérales longilignes présentent une longueur et une largeur suffisantes de sorte à permettre leur positionnement sur le substitut de régénération osseuse et, éventuellement, la membrane de recouvrement et à assurer leur maintien. Typiquement, les extensions latérales longilignes présentent une longueur comprise entre 3 et 15 mm, de préférence entre 3 et 10 mm. Typiquement toujours, les extensions latérales longilignes présentent une largeur comprise entre 1 et 8 mm, de préférence entre 2 et 5 mm.

Avantageusement, et pour faciliter l'adaptation à chaque patient du dispositif destiné à assurer le maintien du substitut de régénération osseuse et, éventuellement, de la membrane de recouvrement, chaque extension latérale longiligne pourra présenter un ou plusieurs points de rupture prédéterminés positionnés dans sa longueur. De la sorte, et lors de la pose du dispositif par le praticien, celui-ci sera en mesure d'utiliser un ou plusieurs de ces points de rupture prédéterminés, de sorte à raccourcir toutes ou partie de ces extensions latérales longilignes et à les adapter à la morphologie du patient. Consécutivement à ce raccourcissement, le praticien pourra, simplement et à l'aide des instruments à sa disposition, éliminer les angles du ou des points de rupture utilisés, de sorte à ne pas risquer de blesser le patient.

Avantageusement encore, et toujours pour faciliter l'adaptation à chaque patient du dispositif destiné à assurer le maintien du substitut de régénération osseuse et, éventuellement, de la membrane de recouvrement, chaque extension latérale longiligne pourra présenter une ou plusieurs amorces qui facilitent son pliage et qui sont positionnées dans sa longueur. De la sorte, et lors de la pose du dispositif par le praticien, celui-ci sera en mesure d'utiliser une ou plusieurs de ces amorces, de sorte à plier toutes ou partie de ces extensions latérales longilignes et à les adapter à la morphologie du patient.

La 3bi) partie centrale de la 3b) au moins une pièce de recouvrement présente deux moitiés distinctes correspondant à une moitié vestibulaire et à une moitié palatine correspondant à la moitié tournée du côté vestibulaire (c'est-à-dire vers l'extérieur de la bouche) et à la moitié tournée du côté palatin (c'est-à-dire vers le palais) respectivement.

Les extensions latérales longilignes dans le prolongement de la moitié vestibulaire de la partie centrale, ou extensions latérales longilignes vestibulaires, présentent une longueur supérieure aux extensions latérales longilignes dans le prolongement de la moitié palatine de la partie centrale ou extensions latérales longilignes palatines.

Cette caractéristique permet de faciliter la différenciation par le praticien du coté vestibulaire et du coté palatin du dispositif destiné à assurer le maintien du substitut de régénération osseuse et, éventuellement, de la membrane de recouvrement.

Typiquement, les extensions latérales longilignes vestibulaires présentent une longueur comprise entre 4 et 10 mm.

On comptera de 2 à 5 extensions latérales longilignes vestibulaires, de préférence de 3 à 4 et, de manière particulièrement préférée, 3 extensions latérales longilignes vestibulaires.

Idéalement, les extensions latérales longilignes vestibulaires présentent, entre elles, un même écartement. Typiquement, ledit écartement est d'au moins 10°, de préférence d'au moins 20° et, de manière particulièrement préférée, d'au moins 25°.

Pour ce qui est des extensions latérales longilignes palatines, leur longueur est typiquement comprise entre 3 et 6 mm.

On comptera de 1 à 4 extensions latérales longilignes palatines, de préférence de 2 à 3 et, de manière particulièrement préférée, 2 extensions latérales longilignes palatines.

Idéalement toujours, les extensions latérales longilignes palatines présentent, entre elles, un même écartement. Typiquement, ledit écartement est d'au moins 10°, de préférence d'au moins 20° et, de manière particulièrement préférée, d'au moins 25°.

Pour présenter ses propriétés particulièrement avantageuses :
- la 3bi) au moins une partie centrale de la 3b) au moins une pièce de recouvrement du 3) au moins un dispositif selon l'invention présente une épaisseur comprise entre 0,3 et 3 mm, de préférence entre 0,5 et 1,5 mm.
- les extensions latérales longilignes présentent une épaisseur comprise entre 0,1 et 1,5mm, de préférence entre 0,3 et 1 mm.

Selon l'invention, la 3b) au moins une pièce de recouvrement forme une seule et même pièce avec le 3a) au moins un pilier externe et, selon un mode de réalisation préféré, le 3c) au moins un pilier intermédiaire. Pour se faire, ceux-ci peuvent être réalisés simultanément en une seule et même pièce ou être couplés à posteriori, notamment par soudage ou sertissage.

Des méthodes de réalisation adaptés pour la fabrication de la 3b) au moins une pièce de recouvrement avec le 3a) au moins un pilier externe et/ou avec le 3c) au moins un pilier intermédiaire en une seule et même pièce sont bien connues de l'homme du métier. A titre d'exemple de telles méthodes, on peut le moulage, le fraisage, ou encore l'impression 3D. Dans le cas d'une telle impression 3D, celle-ci utilise typiquement une poudre métallique dont la mise en forme est assurée par un laser de puissance adaptée.

De même, le soudage ou le sertissage peuvent être effectué par des techniques bien connues de l'homme du métier. Dans le cas où le 3) au moins un dispositif est fait de titane ou d'un alliage de titane, le soudage de ses différents composants peut alors notamment être effectué au laser, par friction ou par bombardement électronique.

En lien avec la 4) au moins une pièce complémentaire du dispositif assurant le maintien de la membrane de recouvrement (6) et qui permet de faciliter la préhension dudit dispositif par le praticien, cette 4) au moins une pièce complémentaire permet donc de faciliter le positionnement dudit 3) au moins un dispositif sur le 1) au moins un pilier d'ancrage dans la bouche du patient par le praticien.

Typiquement, cette pièce complémentaire qui constitue un moyen de préhension prend la forme d'un manchon, qui peut être en plastique, et qui vient se clipser à la partie centrale de la 3) au moins une pièce de recouvrement (8), typiquement dans un logement ménagé au sein de cette partie centrale.

Les exemples qui suivent sont fournis à titre d'illustration et ne sauraient limiter la portée de la présente invention.

### Régénération osseuse d'un défaut osseux de la mâchoire en vue de la pose d'implants dentaires

Dans le cadre d'un défaut osseux au sein de la mâchoire, il est nécessaire de permettre une régénération de ce défaut osseux avant de pouvoir envisager la pose d'implants dentaires.

Pour se faire, une incision de la gencive est effectuée de sorte à accéder à ce défaut osseux dans la mâchoire. Cette incision est effectuée en opérant une élévation du lambeau de gencive consécutif de l'incision.

Le praticien peut alors effectuer un forage au sein de l'os de la mâchoire, idéalement au niveau ou à proximité du défaut osseux. Le praticien effectue alors la pose d'un pied d'ancrage dans le forage effectué précédemment.

Une fois cette pose effectuée, le praticien comble le défaut osseux avec un substitut de régénération osseuse.

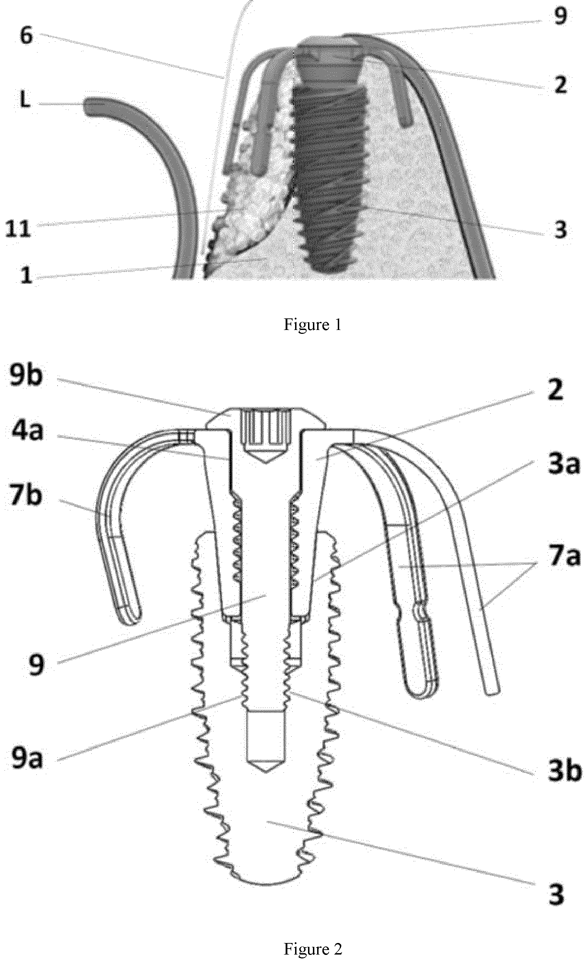

La Figure 1 offre une vue en coupe de la gencive incisée de sorte d'accéder au défaut osseux avec l'élévation d'un lambeau (L) de gencive consécutive de l'incision. On observe également le pied d'ancrage (3) positionné au sein du forage qui a été effectué par le praticien dans l'os (1) de la mâchoire du patient. Autour du pied d'ancrage (3), on visualise le comblement du défaut osseux par le substitut de régénération osseuse (11). Le pied d'ancrage (3) est surplombé du dispositif (2) de maintien du substrat de régénération osseuse (11) avec lequel il est associé par une vis (9). A noter que, dans cette coupe, la membrane de recouvrement (6) est positionnée sur le dispositif (2).

La figure 2 montre une vue en coupe cette fois du système de de régénération osseuse présenté en figure 1, dans lequel on observe le pied d'ancrage (3) et le dispositif (2) avec ses extensions latérales longilignes (7a, 7b). Ce pied d'ancrage (3) et ce dispositif (2) sont associés par le vissage du filetage externe (9a) d'une vis (9) dans le taraudage (3b) de la cavité interne (3a) du pied d'ancrage (3). En effet, une cavité interne (4a) du dispositif (2) de maintien du substitut de régénération osseuse (11) permet le passage de cette vis (9) mais pas de sa tête (9b).

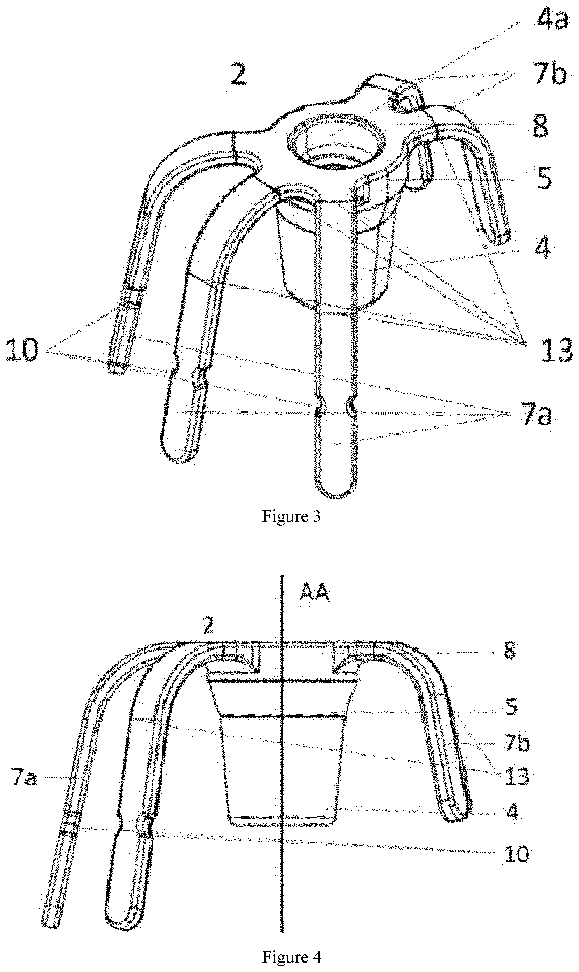

Les figures 3 à 6 montrent des vues schématiques sous différents angles d'un dispositif (2) de maintien du substitut de régénération osseuse. Dans celles-ci, on peut observer le dispositif (2) avec son pilier externe (4), lequel pilier externe (4) est équipé de moyens anti-rotationnels prenant la forme d'un cône-morse de sorte à limiter les mouvements du dispositif (2) une fois son pilier externe (4) mis en place au sein de la cavité complémentaire du pied d'ancrage (cf. figure 2). Ce pilier externe (4) est surplombé d'un pilier intermédiaire (5) de forme conique de sorte à faciliter son passage au sein de la gencive. Au-dessus du pilier intermédiaire (5), se positionne la pièce de recouvrement (8) avec sa partie centrale (12) cylindrique, laquelle partie centrale (12) s'inscrit dans le prolongement du pilier intermédiaire (5). Les extensions latérales longilignes (7a, 7b) de la pièce de recouvrement (8) comprennent deux extensions latérales longilignes palatines (7b), les plus courtes, et trois extensions latérales longilignes vestibulaires (7a), les plus longues. Ces dernières extensions latérales longilignes vestibulaires (7a) comprennent en outre chacune un point de rupture (10) qui permet de faciliter l'ajustement du dispositif (2) par le praticien de sorte à assurer une pose sûre et confortable adaptée au patient. Ces mêmes extensions latérales longilignes vestibulaires (7a) comprennent également deux amorces (13), positionnées dans sa longueur, pour faciliter le pliage par le praticien de ces extensions et l'ajustement du dispositif à la morphologie du patient.

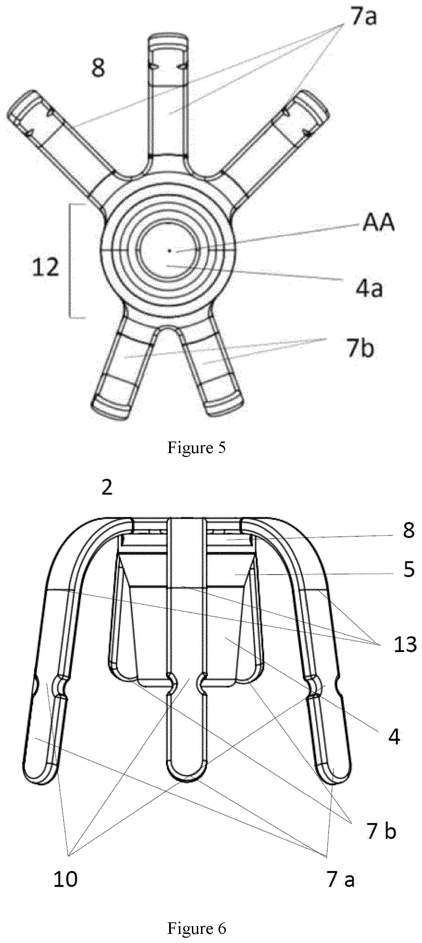

La Figure 4 présente une perspective aérienne de la pièce de recouvrement (8) du dispositif (2), laquelle permet de mieux saisir la disposition spécifique de ses composants. Dans celle-ci, on observe les extensions latérales longilignes (7a, 7b) dans le prolongement de la partie centrale (12) dont l'axe recoupe perpendiculairement l'axe central (AA) (visible sur la figure 6 également). On observe également sur cette vue, la cavité interne (4a) traversant la pièce de recouvrement (8), mais également (non visible sur cette vue) le pilier externe et le pilier intermédiaire.

## Revendications

1. Un kit de régénération osseuse pour un site de défaut osseux dans un os de mâchoire (1) d'un patient qui comprend :
a) un substitut de régénération osseuse (11) permettant le comblement du défaut osseux, et
b) un système de régénération osseuse qui comprend :
1) au moins une vis (9) qui présente une tête (9b) et, à son extrémité distale, un filetage externe (9a), et
2) au moins un dispositif (2) qui comprend :
2a. au moins un pilier externe (4), sur sa face dirigée vers l'os (1), destiné à être fixe dans un pied d'ancrage (3), lequel pilier externe (4) comprend :
2ai. des moyens anti-rotationnels permettant d'inhiber les mouvements relatifs du pilier externe (4) par rapport au pied d'ancrage (3) une fois le pilier externe (4) place dans le pied d'ancrage (3) ;
2aii. une cavité interne (4a) ménagée au sein du pilier externe (4) du dispositif (2) et au sein d'un pilier intermédiaire (5) s'il est présent, laquelle cavité interne (4a) permet le passage de la vis (9), mais pas de sa tête (9b) ; et
2b. au moins une pièce de recouvrement (8), sur sa face opposée à l'os (1) et se plaçant sur le substitut de régénération osseuse (11) et, optionnellement, sur une membrane de recouvrement (6), de sorte à délimiter, en combinaison avec ledit substitut de régénération osseuse (11), le volume de la régénération osseuse au niveau dudit site de défaut osseux, laquelle pièce de recouvrement (8) forme une seule et même pièce avec le pilier extrême (4) et présente :
2bi. une partie centrale (12), dans le prolongement du pilier extrême (4), et
2bii. au moins 3 extensions latérales longilignes (7a, 7b) flexibles et ne comprenant aucun orifice destiné au positionnement d'une vis de fixation, lesquelles extensions sont dans le prolongement de cette partie centrale (12), leur axe recoupant perpendiculairement l'axe central (AA) traversant la partie centrale (12), et
2biii. deux moitiés distinctes pour en faciliter la différenciation par le praticien du côté vestibulaire et du côté palatin, avec une moitié tournée du côté vestibulaire dans le prolongement de laquelle le, ou les, extension(s) latérale(s) longiligne(s) (7a), ou extension(s) latérale(s) longiligne(s) vestibulaire(s) (7a), présentent une longueur supérieure à, ou aux, extension(s) latérale(s) longiligne(s) (7b) dans le prolongement de l'autre moitié de la partie centrale (12) tournée cette fois du côté palatin ou extension(s) latérale(s) longiligne(s) palatine(s) (7b), comprenant de 2 à 5 extensions latérales longilignes vestibulaires (7a) et de 1 à 4 extensions latérales longilignes palatines (7b).
3) au moins un pied d'ancrage (3) pour un implant à venir qui est adapté à une installation de manière endo-osseuse dans l'os de mâchoire (1) au sein même du défaut osseux comprenant une cavité (3a) dans laquelle est ménagé un taraudage (3b) qui peut coopérer avec le filetage (9a) de la vis (9) pour permettre l'assemblage par vissage du pied d'ancrage (3) et du dispositif (2) ; et
4) optionnellement une membrane de recouvrement (6).

2. Le kit selon la revendication 1, **caractérise en ce que** la longueur des extensions latérales longilignes vestibulaires (7a) du dispositif (2) est comprise entre 4 et 10 mm, et celle des extensions latérales longilignes palatines (7b) est comprise entre 3 et 6 mm.

3. Le kit selon l'une quelconque des revendications 1 à 2, **caractérisé en ce que** chaque extension latérale longiligne (7a, 7b) flexible du dispositif (2) présente un ou plusieurs points de rupture (10) prédéterminés positionnés dans sa longueur.

4. Le kit selon l'une quelconque des revendications 1 à 3, **caractérisé en ce que** chaque extension latérale longiligne (7a, 7b) flexible du dispositif (2) présente une ou plusieurs amorces (13) qui facilitent son pliage et qui sont positionnées dans sa longueur.

5. Le kit selon l'une quelconque des revendications 1 à 4, **caractérisé en ce que** le dispositif (2) comprend un pilier intermédiaire (5), entre le pilier extrême (4) et la pièce de recouvrement (8), dont la hauteur est comprise dans l'intervalle allant de 0,5 à 5 mm et qui forme une seule et même pièce avec la pièce de recouvrement (8) et le pilier extrême (4).

6. Le kit selon l'une quelconque des revendications 1 à 5, **caractérisé en ce que** la partie centrale (12) du dispositif (2) présente une épaisseur comprise entre 0,3 et 3 mm **et en ce que** les extensions latérales longilignes (7a, 7b) flexibles présentent une épaisseur comprise entre 0,1 et 1,5mm.

7. Le kit selon l'une quelconque des revendications 1 à 6, **caractérisé en ce que** la partie centrale (12) du dispositif (2) est circulaire.

8. Le kit selon l'une quelconque des revendications 1 à 7, **caractérisé en ce que le** dispositif (2) est réalisé en titane.

9. Le kit selon l'une quelconque des revendications précédentes, **caractérisé en ce que le système** comprend en outre :
4) au moins une membrane de recouvrement (6) qui est de nature résorbable ou non résorbable et dont la surface permet de couvrir au moins la totalité du défaut osseux.

10. Le kit selon l'une quelconque des revendications précédentes, **caractérisé en ce qu'il** comprend en outre :
5) au moins une pièce prenant la forme d'un manchon et qui permet d'en faciliter la préhension par le praticien, laquelle pièce est complémentaire du dispositif (2) et vient se clipser à sa partie centrale (12) dans un logement ménage au sein de celle-ci.

11. Le kit selon l'une quelconque des revendications précédentes, **caractérisé en ce que** les moyens anti-rotationnels équipant le pilier externe (4) prennent la forme d'un cône-morse de sorte à limiter les mouvements du dispositif (2) une fois son pilier externe (4) mis en place au sein de la cavité complémentaire du pied d'ancrage (3).

12. Le kit selon l'une quelconque des revendications précédentes dans lequel le ou les extension(s) latérale(s) longiligne(s) vestibulaire(s) présentent une longueur supérieure à ou aux extension(s) latérale(s) longiligne(s) palatine(s).

13. Le kit selon l'une quelconque des revendications précédentes dans lequel les extensions latérales longilignes vestibulaires présentent, entre elles, un même écartement d'au moins 10°.

14. Le kit selon l'une quelconque des revendications précédentes comprenant de 2 à 3 extensions latérales longilignes palatines présentant, entre elles, un même écartement d'au moins 10°.

## Patentansprüche

1. Knochenregenerationskit für eine Stelle eines Knochendefekts in einem Kieferknochen (1) eines Patienten, umfassend:
a) einen Knochenregenerationsersatz (11), der das Auffüllen des Knochendefekts ermöglicht, und
b) ein Knochenregenerationssystem, umfassend:
1) mindestens eine Schraube (9), die einen Kopf (9b) und an ihrem distale Ende ein Außengewinde (9a) aufweist, und
2) mindestens eine Vorrichtung (2), umfassend:
2a. mindestens eine äußere Säule (4), auf ihrer zum Knochen (1) gerichteten Seite, die dazu bestimmt ist, in einem Verankerungsfuß (3) feststehend zu sein, wobei die äußere Säule (4) umfasst:
2ai. Verdrehsicherungsmittel, die es ermöglichen, die relativen Bewegungen der äußeren Säule (4) in Bezug auf den Verankerungsfuß (3) zu hemmen, nachdem der äußere Säule (4) in den Verankerungsfuß (3) platziert worden ist;
2aii. einen inneren Hohlraum (4a), der innerhalb der äußeren Säule (4) der Vorrichtung (2) und innerhalb einer Zwischensäule (5), falls vorhanden, vorgesehen ist, wobei der innere Hohlraum (4a) den Durchgang der Schraube (9), jedoch nicht ihres Kopfes (9b) ermöglicht; und
2b. mindestens ein Abdeckteil (8), auf ihrer dem Knochen (1) gegenüberliegenden Seite und auf dem Knochenregenerationsersatz (11) und optional auf einer Abdeckmembran (6) platziert, um in Kombination mit dem Knochenregenerationsersatz (11) das Knochenregenerationsvolumen im Bereich der Stelle des Knochendefekts zu begrenzen, wobei das Abdeckteil (8) ein und dasselbe Teil mit der äußersten Säule (4) bildet und aufweist:
2bi. einen Mittelteil (12), in der Verlängerung der äußersten Säule (4), und
2bii. mindestens 3 laterale längliche Verlängerungen (7a, 7b), die flexibel sind und keine Öffnung umfassen, die zum Positionieren einer Befestigungsschraube bestimmt ist, wobei sich die Verlängerungen in der Verlängerung dieses Mittelteils (12) befinden, wobei ihre Achse die Mittelachse (AA), die den Mittelteil (12) durchquert, senkrecht schneidet, und
2biii. zwei getrennte Hälften, um deren Unterscheidung durch den Arzt auf der vestibulären und der palatinalen Seite zu erleichtern, wobei eine Hälfte zur vestibulären Seite gedreht ist, in dessen Verlängerung die laterale(n) längliche(n) Verlängerung(en) (7a), oder laterale(n) längliche(n) vestibuläre(n) Verlängerung(en) (7a) eine Länge aufweisen, die größer als die laterale(n) längliche(n) Verlängerung(en) (7b) in der Verlängerung der anderen Hälfte des Mittelteils (12) sind, die diesmal zur palatinale Seite oder laterale(n) längliche(n) palatinale(n) Verlängerung(en) (7b) gedreht ist, die 2 bis 5 laterale längliche vestibuläre Verlängerungen (7a) und 1 bis 4 laterale längliche palatinale Verlängerungen (7b) umfasst.
3) mindestens einen Verankerungsfuß (3) für ein künftiges Implantat, das für eine enossale Installation im Kieferknochen (1) innerhalb des Knochendefekts angepasst ist, umfassend einen Hohlraum (3a), in dem ein Innengewinde (3b) vorgesehen ist, das mit dem Gewinde (9a) der Schraube (9) zusammenwirken kann, um die Schraubverbindung des Verankerungsfußes (3) und der Vorrichtung (2) zu ermöglichen; und
4) optional eine Abdeckmembran (6).

2. Kit nach Anspruch 1, **dadurch gekennzeichnet, dass** die Länge der lateralen länglichen vestibulären Verlängerungen (7a) der Vorrichtung (2) zwischen 4 und 10 mm umfasst, und jene der lateralen länglichen palatinalen Verlängerungen (7b) zwischen 3 und 6 mm umfasst.

3. Kit nach einem der Ansprüche 1 bis 2, **dadurch gekennzeichnet, dass** jede flexible laterale längliche Verlängerung (7a, 7b) der Vorrichtung (2) eine oder mehrere in ihrer Länge positionierte vorbestimmte Bruchpunkte (10) aufweist.

4. Kit nach einem der Ansprüche 1 bis 3, **dadurch gekennzeichnet, dass** jede flexible laterale längliche Verlängerung (7a, 7b) der Vorrichtung (2) einen oder mehrere Ansätze (13) aufweist, die deren Biegen erleichtern und die in ihrer Länge positioniert sind.

5. Kit nach einem der Ansprüche 1 bis 4, **dadurch gekennzeichnet, dass die** Vorrichtung (2) eine Zwischensäule (5) zwischen der äußersten Säule (4) und dem Abdeckteil (8) umfasst, deren Höhe im Intervall von 0,5 bis 5 mm umfasst, und die mit dem Abdeckteil (8) und der äußersten Säule (4) ein und dasselbe Teil bildet.

6. Kit nach einem der Ansprüche 1 bis 5, **dadurch gekennzeichnet, dass** der Mitteleil (12) der Vorrichtung (2) eine Dicke aufweist, die zwischen 0,3 und 3 mm umfasst, **und dadurch, dass** die flexiblen lateralen länglichen Verlängerungen (7a, 7b) eine Dicke aufweisen, die zwischen 0,1 und 1,5 mm umfasst.

7. Kit nach einem der Ansprüche 1 bis 6, **dadurch gekennzeichnet, dass** der Mittelteil (12) der Vorrichtung (2) kreisförmig ist.

8. Kit nach einem der Ansprüche 1 bis 7, **dadurch gekennzeichnet, dass** die Vorrichtung (2) aus Titan hergestellt ist.

9. Kit nach einem der vorstehenden Ansprüche, **dadurch gekennzeichnet, dass das System** weiter umfasst:
4 mindestens eine Abdeckmembran (6), die resorbierbar oder nicht resorbierbar ist und deren Oberfläche es ermöglicht, mindestens den gesamten Knochendefekt abzudecken.

10. Kit nach einem der vorstehenden Ansprüche, **dadurch gekennzeichnet, dass** er weiter umfasst:
5) mindestens ein Teil, das die Form einer Hülse annimmt, und deren Ergreifen durch den Arzt erleichtert, wobei das Teil ergänzend zur Vorrichtung (2) ist und an seinem Mittelteil (12) in eine Aufnahme einrastet, die innerhalb derselben vorgesehen ist.

11. Kit nach einem der vorstehenden Ansprüche, **dadurch gekennzeichnet, dass** die Verdrehsicherungsmittel, welche die äußere Säule (4) ausstatten, die Form eines Morsekonus annehmen, um die Bewegungen der Vorrichtung (2) zu begrenzen, sobald ihre äußere Säule (4) im ergänzenden Hohlraum des Verankerungsfußes (3) platziert worden ist.

12. Kit nach einem der vorstehenden Ansprüche, wobei die laterale(n) längliche(n) vestibuläre(n) Verlängerung(en) eine Länge aufweist/aufweisen, die länger ist/sind als die laterale(n) längliche(n) palatinale(n) Verlängerung(en).

13. Kit nach einem der vorstehenden Ansprüche, wobei die vestibulären lateralen länglichen Verlängerungen untereinander einen gleichen Abstand von mindestens 10° aufweisen.

14. Kit nach einem der vorstehenden Ansprüche, der 2 bis 3 palatinale laterale längliche Verlängerungen umfasst, die untereinander einen gleichen Abstand von mindestens 10° aufweisen.

## Claims

1. Bone regeneration kit for a bone defect site in a jaw bone (1) of a patient, which comprises:
a) a bone regeneration substitute (11) for filling the bone defect, and
b) a bone regeneration system, which comprises:
1) at least one screw (9) which has a head (9b) and, at its distal end, an external thread (9a), and
2) at least one device (2), which comprises:
2nd. at least one outer abutment (4), on its face oriented towards the bone (1), intended to be fixed in an anchor base (3), said outer abutment (4) comprising:
2ai. anti-rotational means for inhibiting relative movements of the outer abutment (4) with respect to the anchor base (3) once the outer abutment (4) is placed in the anchor base (3);
2aii. an inner cavity (4a) arranged within the outer abutment (4) of the device (2) and within an intermediate abutment (5) if it is present, said inner cavity (4a) allowing passage of the screw (9), but not of its head (9b); and
2b. at least one covering part (8), on its face opposite the bone (1) and placed on the bone regeneration substitute (11) and, optionally, on a covering membrane (6), so as to delimit, in combination with said bone regeneration substitute (11), the volume of bone regeneration at said bone defect site, said covering part (8) forming one piece with the end abutment (4) and having:
2bi. a central portion (12), in line with the end abutment (4), and
2bii. at least 3 flexible elongated lateral extensions (7a, 7b), not comprising any hole intended for positioning a fastening screw, said extensions being in line with this central portion (12), their axis intersecting perpendicularly the central axis (AA) passing through the central portion (12), and
2biii. two separate halves in order to facilitate differentiation by the practitioner of the vestibular side and the palatal side, with one half oriented towards the vestibular side in line with which the elongated lateral extension(s) (7a), or vestibular elongated lateral extension(s) (7a), have a length greater than the elongated lateral extension(s) (7b) in line with the other half of the central portion (12) this time oriented towards the palatal side or palatal elongated lateral extension(s) (7b), comprising from 2 to 5 vestibular elongated lateral extensions (7a) and from 1 to 4 palatal elongated lateral extensions (7b).
3) at least one anchor base (3) for an upcoming implant which is adapted for endosteal installation in the jaw bone (1) within the same bone defect comprising a cavity (3a) in which an internal thread (3b) is formed, which can cooperate with the external thread (9a) of the screw (9) to allow assembly of the anchor base (3) and the device (2) by screwing; and
4) optionally a covering membrane (6).

2. Kit according to claim 1, **characterised in that** the length of the vestibular elongated lateral extensions (7a) of the device (2) is between 4 and 10 mm, and that of the palatal elongated lateral extensions (7b) is between 3 and 6 mm.

3. Kit according to any one of claims 1 to 2, **characterised in that** each flexible elongated lateral extension (7a, 7b) of the device (2) has one or more predetermined fracture points (10) positioned lengthwise.

4. Kit according to any one of claims 1 to 3, **characterised in that** each flexible elongated lateral extension (7a, 7b) of the device (2) has one or more primers (13) which facilitate its bending and which are positioned lengthwise.

5. Kit according to any one of claims 1 to 4, **characterised in that the** device (2) comprises an intermediate abutment (5), between the end abutment (4) and the covering part (8), the height of which is comprised in the range from 0.5 to 5 mm and which forms one piece with the covering part (8) and the end abutment (4).

6. Kit according to any one of claims 1 to 5, **characterised in that** the central portion (12) of the device (2) has a thickness between 0.3 and 3 mm **and in that** the flexible elongated lateral extensions (7a, 7b) have a thickness between 0.1 and 1.5 mm.

7. Kit according to any one of claims 1 to 6, **characterised in that** the central portion (12) of the device (2) is circular.

8. Kit according to any one of claims 1 to 7, **characterised in that** the device (2) is made of titanium.

9. Kit according to any one of the preceding claims, **characterised in that the system** further comprises:
4 at least one covering membrane (6) which is absorbable or non-absorbable in nature and the surface of which allows covering at least the entirety of the bone defect.

10. Kit according to any one of the preceding claims, **characterised in that** it further comprises:
5) at least one part taking the form of a sleeve and which allows easier gripping thereof by the practitioner, said part being complementary with the device (2) and clipping onto its central portion (12) in a housing arranged therein.

11. Kit according to any one of the preceding claims, **characterised in that** the anti-rotational means equipping the outer abutment (4) take the form of a Morse taper so as to limit movements of the device (2) once its outer abutment (4) is placed within the complementary cavity of the anchor base (3).

12. Kit according to any one of the preceding claims wherein the vestibular elongated lateral extension(s) have a length greater than the palatal elongated lateral extension(s).

13. Kit according to any one of the preceding claims, wherein the vestibular elongated lateral extensions have, between them, the same spacing of at least 10°.

14. Kit according to any one of the preceding claims comprising from 2 to 3 palatal elongated lateral extensions having, between them, the same spacing of at least 10°.
